(19) Europäisches Patentamt | European Patent Office | Office européen des brevets

(11) **EP 3 742 979 B1**

(12) **EUROPEAN PATENT SPECIFICATION**

(45) Date of publication and mention
of the grant of the patent:
**19.10.2022 Bulletin 2022/42**

(21) Application number: **19701514.2**

(22) Date of filing: **23.01.2019**

(51) International Patent Classification (IPC):
**A61B 8/06** *(2006.01)*     **A61B 8/08** *(2006.01)*
**A61B 8/00** *(2006.01)*

(52) Cooperative Patent Classification (CPC):
**A61B 8/0891; A61B 8/06; A61B 8/0808;
A61B 8/461; A61B 8/488; A61B 8/52;** A61B 8/4218

(86) International application number:
**PCT/EP2019/051593**

(87) International publication number:
**WO 2019/145343 (01.08.2019 Gazette 2019/31)**

(54) **GUIDED-TRANSCRANIAL ULTRASOUND IMAGING USING NEURAL NETWORKS AND ASSOCIATED DEVICES, SYSTEMS, AND METHODS**

GEFÜHRTE TRANSKRANIALE ULTRASCHALLBILDGEBUNG UNTER VERWENDUNG NEURONALER NETZWERKE UND ZUGEHÖRIGE VORRICHTUNGEN, SYSTEME UND VERFAHREN

IMAGERIE ULTRASONORE TRANSCRÂNIENNE GUIDÉE À L'AIDE DE RÉSEAUX NEURONAUX ET DISPOSITIFS, SYSTÈMES ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2018 US 201862621175 P**

(43) Date of publication of application:
**02.12.2020 Bulletin 2020/49**

(73) Proprietor: **Koninklijke Philips N.V.
5656 AG Eindhoven (NL)**

(72) Inventors:
• **ERRICO, Claudia
5656 AE Eindhoven (NL)**

• **SUTTON, Jonathan, Thomas
5656 AE Eindhoven (NL)**
• **SWISHER, Christine
5656 AE Eindhoven (NL)**
• **WANG, Haibo
5656 AE Eindhoven (NL)**

(74) Representative: **Philips Intellectual Property & Standards
High Tech Campus 52
5656 AG Eindhoven (NL)**

(56) References cited:
**US-A1- 2002 103 436     US-A1- 2005 015 009
US-A1- 2015 151 142**

**Description**

## TECHNICAL FIELD

The present disclosure relates generally to ultrasound imaging, in particular, to applying neural networks to guide a user in aligning an ultrasound imaging component to a desired imaging plane during a transcranial examination.

## BACKGROUND

Cerebrovascular hemodynamic measurements can be used to diagnose and monitor various cerebrovascular conditions in adult and pediatric populations. Radio-opaque computed tomography (CT) tracer-based techniques and magnetic resonance imaging (MRI) contrast agent-based techniques are commonly used to obtain cerebrovascular hemodynamic measurements. However, radio-opaque CT tracer-based and MRI contrast agent-based techniques may not provide a high enough temporal resolution for assessing hemodynamics. In addition, the radio-opaque CT tracer-based and the MRI contrast agent-based techniques may require extensive equipment and setup and can be expensive.

Another approach to measuring blood flow in intracranial arteries is to employ transcranial Doppler (TCD) ultrasound. TCD ultrasound is a non-invasive ultrasound imaging technique that can be used for point-of-care testing and diagnosis. During TCD monitoring, ultrasound waves are transmitted through a patient's skull and reflect off blood flow within the brain. The frequency shift in the echo signals allows estimation of the blood flow and detection of various cerebrovascular conditions. For example, TCD can detect and monitor intracranial aneurysms, patent foramen ovale, vasospasm, stenosis, brain death, shunts, and microemboli in surgical or ambulatory settings without generating radiation.

While TCD ultrasound can provide cerebrovascular hemodynamic measurements with a sufficiently high temporal resolution at a relatively low cost, consistent TCD measurements are difficult to obtain. Attenuation and aberration of the skull bones, and variability and tortuosity of perforating cerebral vessels require highly trained or experienced operators. For example, an accurate TCD exam may require an operator to have knowledge and understanding of cerebrovasculature topologies, cerebrovasculature patterns, cerebrovasculature variations, and/or Doppler ultrasound techniques. One challenge in transcranial ultrasound imaging is the blurring and signal absorption that occur due to skull bones. These acoustic effects bend ultrasound beams, making vascular flow patterns difficult to recognize. In these cases, expert users may rely on hallmark topologies and vessel bifurcations near the Circle of Willis (CoW) to obtain blood flow measurements. Nevertheless, poor image quality may prevent accurate TCD-based examinations. As a result, the scope of using TCD as a clinical tool may be limited. In addition, TCD measurements may be subject to inter-operator variability even among experienced operators.

One approach to assisting a user to perform TCD ultrasound imaging is to provide imaging feedback by displaying a depth projection of power Doppler signals while the user searches for a middle cerebral artery (MCA) in a patient's brain. While the imaging feedback may allow a user to monitor hemodynamic with a higher consistency or accuracy, the feedbackbased approach is limited to proximal MCA examinations and may be subjected to variation in the tortuosity of M1 and M2 branches of the MCA.

US 2002/0103436 discloses a system for automatic manipulation of a transcranial Doppler probe device comprising a cylindrical probe housing having an inner electrical wiring, an ultrasound transducer with cable affixed on the probe cylindrical base having a coil, said probe cylindrical base placed within the cylindrical probe housing, a system of roller balls, a system software program and microprocessor for controlling the probe position, and a removable handle attached to the said probe cylindrical base. The system software "learns" probe angulations after initial manual manipulations and then performs cerebral vessel insonation using electromotive force independent of an operator.

## SUMMARY

While existing procedures for using TCD ultrasound imaging to assess cerebrovascular conditions have proved useful for clinical procedures, there remains a clinical need for improved systems and techniques for providing efficient, accurate, and automatic procedures for aligning an ultrasound imaging component to a desired imaging plane for a transcranial examination. Embodiments of the present disclosure provide mechanisms for using a deep learning network to guide a user during a TCD examination. For example, an ultrasound imaging component may capture an image of flow within cerebral blood vessels (e.g., in a color-Doppler image). The captured image can be feed into a convolutional neural network (CNN) that is trained to identify a current imaging plane of the ultrasound imaging component or a current vascular location captured by the image within a cerebrovascular atlas (e.g., a known brain vessel topography). The target vascular location or the target imaging plane for a transcranial examination in the cerebrovascular atlas may be known (e.g., the location of an MCA for an MCA examination can be predetermined). Thus, a set of motion control parameters for aligning the ultrasound imaging component to the target imaging plane can be computed based on a geometric distance or angulation calculation between the current imaging plane and the target imaging plane. The disclosed embodiments can provide instructions to guide the alignment of the ultrasound imaging component to the target imaging plane based on the motion control parameters. The disclosed embodiments can provide a graphical view including an overlay of the current imaging plane, the target imaging plane, and/or the imaged blood vessels on top of a cerebrovascular atlas. The disclosed embodiments can provide a graphical view including a virtual view of the target vessel region, which may be outside a current field-of-view of the ultrasound imaging component.

In one embodiment, a medical ultrasound imaging system is provided. The system includes an interface in communication with an ultrasound imaging component and configured to receive a first image representative of blood vessels of a brain of a patient while the ultrasound imaging component is positioned at a first imaging position with respect to the patient; and a processing component in communication with the interface and configured to apply a convolutional network (CNN) to the first image to produce a motion control configuration for repositioning the ultrasound imaging component from the first imaging position to a second imaging position associated with a transcranial examination, the CNN trained based on at least a known blood vessel topography.

In some embodiments, the processing component is further configured to determine Doppler information representative of blood flow within the blood vessels of the patient's brain based on data associated with the first image, and wherein the CNN is applied to the Doppler information. In some embodiments, the processing component is further configured to determine connectivity information associated with the blood vessels of the patient's brain based on the Doppler information, and determine a covariance matrix based on the connectivity information, and wherein the CNN is applied to the covariance matrix. In some embodiments, the connectivity information includes coordinates corresponding to vascular locations along the blood vessels of the patient's brain. In some embodiments, the processing component is further configured to apply the CNN to the Doppler information to determine an imaging plane corresponding to the first imaging position within the known blood vessel topography; and determine the motion control configuration based on the imaging plane and a target imaging plane associated with the transcranial examination within the known blood vessel topography. In some embodiments, the processing component is further configured to apply the CNN to the Doppler information to determine a feature vector representative of the blood vessels of the patient's brain; and determine the imaging plane within the known blood vessel topography based on a comparison of the feature vector against the known blood vessel topography. In some embodiments, the CNN is further trained based on at least a covariance matrix determined based on connectivity information of the known blood vessel topography and a weighting function associated with the transcranial examination, and wherein the connectivity information includes coordinates corresponding to vascular locations along blood vessels indicated in the known blood vessel topography. In some embodiments, the motion control configuration includes at least one of a translation or a rotation of the ultrasound imaging component. In some embodiments, the system further comprises a user interface in communication with the processing component, the user interface configured to receive a selection of at least one of a type of the transcranial examination or a target vascular location associated with the transcranial examination, wherein the processing component is further configured to determine the second imaging position based on the selection. In some embodiments, the system further comprises a display in communication with the processing component, the display configured to display an instruction, based on the motion control configuration, for operating the ultrasound imaging component such that the ultrasound imaging component is repositioned to the second imaging position. In some embodiments, the system further comprises a display in communication with the processing component, the display configured to display a graphical view including an overlay of at least one of a first imaging plane associated with the first imaging position, a second imaging plane associated with the second imaging position, or the blood vessels of the patient's brain on top of the known blood vessel topography. In some embodiments, the system further comprises a display in communication with the processing component, the display configured to display a graphical view including an overlay of an expected view of blood vessels of the patient's brain associated with the second imaging position on top of the known blood vessel topography.

In one embodiment, a method of medical ultrasound imaging is provided. The method includes receiving, from an ultrasound imaging component, a first image representative of blood vessels of a brain of a patient while the ultrasound imaging component is positioned at a first imaging position with respect to the patient; and applying a convolutional network (CNN) to the first image to produce a motion control configuration for repositioning the ultrasound imaging component from the first imaging position to a second imaging position associated with a transcranial examination, the CNN trained based on at least a known blood vessel topography.

In some embodiments, the method further comprises determining Doppler information representative of blood flow within the blood vessels of the patient's brain based on data associated with the first image, wherein the CNN is applied to the Doppler information. In some embodiments, the method further comprises determining connectivity information associated with the blood vessels of the patient's brain based on the Doppler information, the connectivity information including coordinates corresponding to vascular locations along the blood vessels of the patient's brain; and determining a covariance matrix based on the connectivity information, and wherein the CNN is applied to the covariance matrix. In some embodiments, the method further comprises applying the CNN to the Doppler information to determine a feature vector representative of the blood vessels of the patient's brain; determining an imaging plane within the known blood vessel topography based on a comparison of the feature vector against the known blood vessel topography; and determining the motion control configuration based on the imaging plane and a target imaging plane associated with the transcranial examination within the known blood vessel topography, the motion control configuration including at least one of a translation or a rotation for operating the ultrasound imaging component. In some embodiments, the method further comprises receiving a selection of at least one of a type of the transcranial examination or a target vascular location associated with the transcranial examination; and determining the second imaging position based on the selection. In some embodiments, the method further comprises transmitting an instruction to at least one of a display or a robotic system, based on the motion control configuration, for operating the ultrasound imaging component such that the ultrasound imaging component is repositioned to the second imaging position, the instruction including at least one of a translation or a rotation of the ultrasound imaging component. In some embodiments, the method further comprises displaying a graphical view including an overlay of at least one of a first imaging plane associated with the first imaging position, a second imaging plane associated with the second imaging position, or the blood vessels of the patient's brain on top of the known blood vessel topography. In some embodiments, the method further comprises displaying a graphical view including an overlay of an expected view of blood vessels of the patient's brain associated with the second imaging position on top of the known blood vessel topography.

Additional aspects, features, and advantages of the present disclosure will become apparent from the following detailed description.

## BRIEF DESCRIPTION OF THE DRAWINGS

Illustrative embodiments of the present disclosure will be described with reference to the accompanying drawings, of which:

FIG. 1 is a schematic diagram of a medical ultrasound imaging system for transcranial examinations, according to aspects of the present disclosure.

FIG. 2 is a schematic diagram illustrating a vasculature of a patient's brain, according to aspects of the present disclosure.

FIG. 3 is a schematic diagram illustrating a graphical representation of a portion of a vasculature of a patient's brain, according to aspects of the present disclosure.

FIG. 4 is a schematic diagram illustrating a scheme for guiding an ultrasound imaging component to a desired imaging plane for a transcranial examination, according to aspects of the present disclosure.

FIG. 5 illustrates an example of two-dimensional (2D) Doppler imaging, according to aspects of the present disclosure.

FIG. 6 illustrates an example of three-dimensional (3D) Doppler imaging, according to aspects of the present disclosure.

FIG. 7 is a schematic diagram illustrating a configuration for a convolutional neural network (CNN), according to aspects of the present disclosure.

FIG. 8 is a schematic diagram illustrating a scheme for generating a covariance matrix from a cerebrovascular atlas, according to aspects of the present disclosure.

FIG. 9 is a schematic diagram illustrating a display for guiding transcranial ultrasound imaging, according to aspects of the present disclosure.

FIG. 10 is a flow diagram of a method of applying a CNN to guide an ultrasound imaging component to a desired imaging plane for a transcranial examination, according to aspects of the disclosure.

## DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

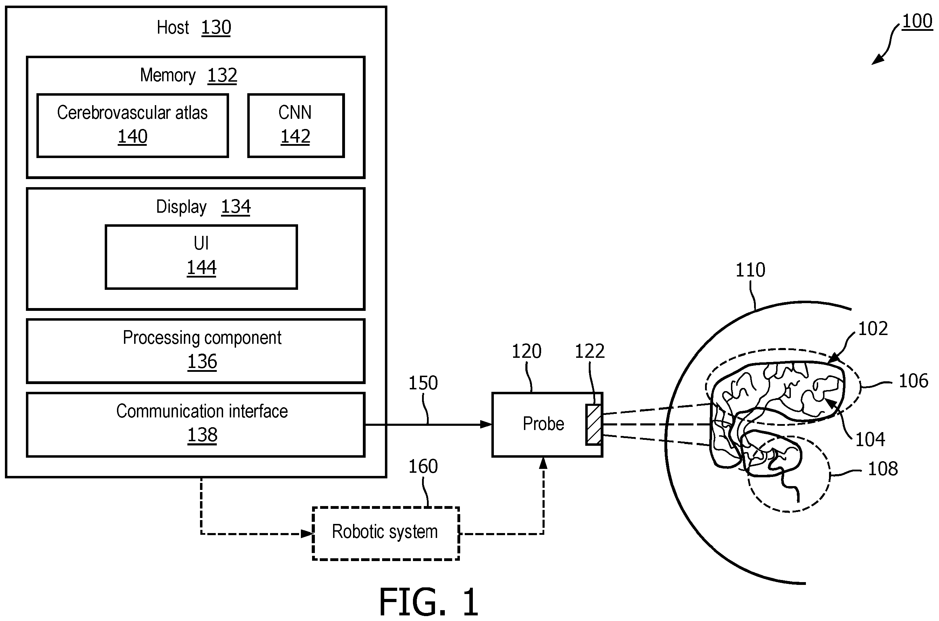

FIG. 1 is a schematic diagram of a medical imaging system for transcranial examinations, according to aspects of the present disclosure. The system 100 includes a host 130 and an imaging probe 120 in communication with each other. At a high level, the probe 120 can be placed in contact with the patient's head 110 to capture images of the patient's brain 102, and/or blood vessels 104 within the patient's brain 102 and the host 130 can provide a user with instructions to reposition the probe 120 to a desired location for imaging a region of interest for a particular transcranial examination. For instance, a transcranial examination may measure blood flow within blood vessels around the Circle of Willis (CoW) such as an anterior cerebral artery (ACA), an internal carotid artery (ICA), a middle cerebral artery (MCA), a posterior cerebral artery (PCA), a posterior communicating artery, a basilar artery (BA) and/or blood vessels fed by corresponding arteries. The arteries associated with the CoW are described in greater detail herein. In some instances, a transcranial examination may also measure blood flow within other arteries and/or veins in any location of a patient's head 110 or a patient's brain 102. The system 100 may be an ultrasound imaging system and the probe 120 may be an external ultrasound imaging probe.

The probe 120 may include an imaging component 122 including one or more ultrasound sensors or transducer elements. The transducer elements may emit ultrasonic energy towards an anatomy (e.g., the head 110) of a patient. The ultrasonic energy is reflected by the vasculatures and the tissues of the patient's brain and the skull bones of the patient. The ultrasound transducer elements may receive the reflected ultrasound signals. In some embodiments, the probe 120 may include an internal or integrated processing component that can process the ultrasound echo signals locally to generate image signals representative of the patient's anatomy under imaging. The ultrasound transducer element(s) can be arranged to provide 2D images or 3D images of the patient's anatomy. The probe 120 may be configured to perform duplex-mode ultrasound imaging with both B-mode imaging and color-Doppler flow measurements. A user may place the probe 120 at various locations on an external surface of a patient's head 110 to carry out a transcranial examination.

For instance, the probe 120 may be placed at a certain location on a patient's head 119. The location may be chosen based on bone thickness and bone composition of the patient's head 110. For example, compact bone has less air, and thus may reflect sound waves less compared to trabecular bone. The locations that allow efficient ultrasound transmission for transcranial examinations may be referred to as transcranial windows or acoustic windows. Transcranial windows that are commonly used to examine the six major cerebral arteries (e.g., the ACA, the ICA, the MCA, the PCA, the posterior communicating artery, and the BA) may include a temporal window, a submandibular window, and a suboccipital window. For instance, the temporal window may be used for examining the ACA, the MCA, the ICA, the PCA, the posterior communicating artery, and neighboring vessels fed by corresponding arteries. Alternatively, the suboccipital transcranial window may be used for examining the BA or the submandibular transcranial window may be used for examining the ICA. A user may maneuver the probe 120 through translations and/or rotations of the probe 120 to reach a target location corresponding to a transcranial window of a selected transcranial examination. The host 130 may provide guidance to a user during TCD imaging to facilitate correct acquisition of flow dynamics within the vasculature within the patient's head 110 or the patient's brain 102. For example, the TCD imaging can be transcranial color-Doppler (TCCD) imaging.

The host 130 may include a memory 132, a display 134, a processing component 136, and a communication interface 138. The processing component 136 may be coupled to and in communication with the memory 132, the display 134, and the communication interface 138. The host 130 may be a computer work station, a mobile phone, a tablet, or any suitable computing device.

The memory 132 may include a cache memory (e.g., a cache memory of the processing component 136), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory. The memory 132 may be configured to store a cerebrovascular atlas 140 and one or more CNNs 142.

The cerebrovascular atlas 140 may be a 3D model that describes a generalized or personalized arrangement of blood vessels within human brains. While the variability and the tortuosity of the blood vessels in the lateral regions of human brains can cause blood vessel identification to become difficult, the CoW is bilateral and symmetric, exhibiting hallmark morphologies, connectivity patterns, and flow dynamics. Thus, the predictable portions (e.g., the CoW) of blood vessels within human brains can be used to construct a cerebrovascular atlas 140. For example, a cerebrovascular atlas 140 may include connectivity, topology, and location information of major cerebral vessels (e.g., the MCA, the ICA, the ACA, the PCA, and the BA) and associated with blood vessels with respect to each other and with respect to the bone structure in a human skull. The locations and/or topologies of the major vessels are relatively predictable and similar across patients, whereas the locations and/or topologies of the smaller vessels, for example, distal to the major vessels, may vary among different patients. For example, FIG. 1 illustrates a portion 108 of the vasculature within the patient's head 110 including an ACA where the location or arrangement of the ACA may be relatively predictable across patients and a portion 106 including vessels distal to the ACA where the locations or arrangements may vary for different patients. In some embodiments, the memory 132 may store multiple cerebrovascular atlases 140. For example, the blood vessel topographies in the cerebrovascular atlases 140 may be generated based on imaging data of patients' brains collected from clinical studies and/or imaging data previously captured from a corresponding patient under examination. The atlas 140 can be configured to store empirically known data about blood vessels in the patient head, brain, neck, and/or other anatomy, including blood vessel structure, relationship, connections, blood flow patterns, geometry, location from one or more imaging windows, etc. A patient undergoing a current examination may or may not be a part of the plurality of patients upon which the atlas 140 is based. In some embodiments, patient-specific blood vessel data associated with the patient undergoing the current examination is utilized as the atlas 140, e.g., from earlier imaging of the patient's brain. The patient-specific blood vessel data can be utilized in lieu of or in addition to reference data from a plurality of other patients.

The CNN 142 may be trained to identify a location of a blood vessel imaged by the probe 120 with respect to the cerebrovascular atlas 140. In some embodiments, the memory 132 may store multiple CNNs142. For example, the CNNs 142 may include a predictive CNN for identifying a current imaging plane to provide guidance to a user in aligning the probe 120 to reach a target or desired imaging plane and a qualifying CNN for qualifying the identification provided by the predictive CNN.

The processing component 136 may include a central processing unit (CPU), a digital signal processor (DSP), an application specific integrated circuit (ASIC), a controller, a field programmable gate array (FPGA) device, another hardware device, a firmware device, or any combination thereof configured to perform the operations described herein. The processing component 136 may also be implemented as a combination of computing devices, e.g., a combination of a DSP and a microprocessor, a plurality of microprocessors, one or more microprocessors in conjunction with a DSP core, or any other such configuration.

In an embodiment, the processing component 136 is configured to receive an image of the patient's head 110 from the probe 120. The processing component 136 can determine Doppler information (e.g., blood flow within the vessels 104) from the image and determine a graphical representation of the blood vessels 104 based on the Doppler information. For example, the graphical representation may include spatial coordinates that describe the locations along segments of the blood vessels and the connectivity of the blood vessels. In some instances, the graphical representation may be in the form of a connectivity graph or a tree diagram. The processing component 136 can determine a current location of the probe 120 with respect to the vasculature of the patient based on the connectivity information and determine a control configuration (e.g., including translation and/or rotation parameters) for repositioning the probe 120 to a target location or a target imaging plane for obtaining an image for a particular transcranial examination.

In an embodiment, the processing component 136 may apply the CNN 142 to the Doppler information and the CNN 142 may identify a current location of the probe 120 within the cerebrovascular atlas 140. The processing component 136 may determine a translation and/or a rotation that may be required to reposition the probe 120 to the target location.

In an embodiment, the processing component 136 is configured to train the CNN 142 for aligning the imaging component 122 to target image planes based on one or more cerebrovascular atlases 140. In an embodiment, the processing component 136 is configured to apply the CNN 142 in a clinical setting to determine motion control parameters to align the probe 120 to a patient for a particular transcranial examination. For instance, the imaging component 122 is aligned to obtain an image of an ACA of the patient for a transcranial examination. Mechanisms for mapping Doppler information into a graphical representation, training the CNN 142, and applying the CNN 142 are described in greater detail herein.

In some embodiments, the memory 132 may include a non-transitory computer-readable medium. The memory 132 may store instructions that, when executed by the processing component 136, cause the processing component 136 to perform the operations described herein with references to the CNN training and/or CNN application in connection with embodiments of the present disclosure. Instructions may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer readable

The display 134 may include a computer screen or any suitable display for displaying a user interface (UI) 144. The UI 144 may include a graphical representation or view of the probe 120. The UI 144 may include visual indicators indicating a translation and/or rotation of the probe 120. The UI 144 may include a graphical view including an overlay of a current image taken by the probe 120 on top of the cerebrovascular atlas 140. The graphical view may additionally include an overlay of an expected view of the patient's vasculatures or vessels 104 at the target location on top of the cerebrovascular atlas 140. While the display 134 is shown as an integrated component of the host 130, in some embodiments, the display 134 may be external to the host 130 and in communication with the host 130 via the communication interface 138. For instance, the display 134 may include a standalone display, an augmented reality glasses, or a mobile phone.

The communication interface 138 may be configured to communicate with the imaging component 122 of the probe 120 via a communication link 150. For example, the host 130 may send controls to control the transmission and receptions of ultrasound transducer elements (e.g., for beamforming) and may receive acquired images from the probe 120 via the communication link 150. The communication link 150 may include a wireless link and/or a wired link. Examples of a wireless link may include a low-power Bluetooth® wireless link, an Institute of Electrical and Electronics Engineers (IEEE) 802.11 (WiFi) link, or any suitable wireless link. Examples of a wired link may include a universal serial bus (USB) link or any suitable wired link.

In some embodiments, the communication interface 138 may be further configured to receive user inputs, for example, via a keyboard, a mouse, or a touchscreen. The UI 144 may update a certain display or view based on the user input. The UI 144 is described in greater detail herein.

In some embodiments, the system 100 may further include a robotic system 160 in communication with the communication interface 138 and the probe 120. The robotic system 160 may include electrical and/or mechanical components, such as motors, rollers, and gears, configured to reposition the probe 120. In such embodiments, the processing component 136 can be configured to send the motion control parameters to the robotic system 150, for example, via the communication interface 138. The robotic system 160 may automatically align the probe 120 to a patient for a particular transcranial examination based on the motion control parameters. For example, the robotic system 150 could automatically align the probe without manual repositioning by the user.

While the system 100 is illustrated with an ultrasound imaging probe 120, the system 100 may be configured to automatically align any suitable imaging component 122 to a patient for a clinical procedure. The imaging component 122 may provide any suitable imaging modalities. Example of imaging modalities may include optical imaging, optical coherence tomography (OCT), radiographic imaging, x-ray imaging, angiography, fluoroscopy, computed tomography (CT), magnetic resonance imaging (MRI), elastography, etc.

In some other embodiments, the system 100 may include any suitable sensing component, including a pressure sensor, a flow sensor, a temperature sensor, an optical fiber, a reflector, a mirror, a prism, an ablation element, a radio frequency (RF) electrode, a conductor, and/or combinations thereof for performing a clinical or therapy procedure, where images of a patient's anatomy receiving the procedure may be captured by the imaging component 122 before, during, and/or after the procedure.

Generally, the system 100, the probe 120, and/or other devices described herein can be utilized to examine any suitable anatomy of a patient body. In some instances, the probe 120 can be positioned outside of a patient's body to examine the anatomy and/or lumen inside of the patient's body. For the anatomy and/or lumen may represent fluid filled or surrounded structures, both natural and man-made. For example, a probe of the present disclosure can be positioned on a surface of a patient's head to obtain blood flow measurements within the patient's brain. In some embodiments, a probe of the present disclosure may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves, chambers or other parts of the heart, and/or other systems of the body. The anatomy and/or lumen inside of the patient's body may be a blood vessel, as an artery or a vein of a patient's vascular system, including cerebral vasculature, cardiac vasculature, peripheral vasculature, neural vasculature, renal vasculature, and/or or any other suitable lumen inside the body.

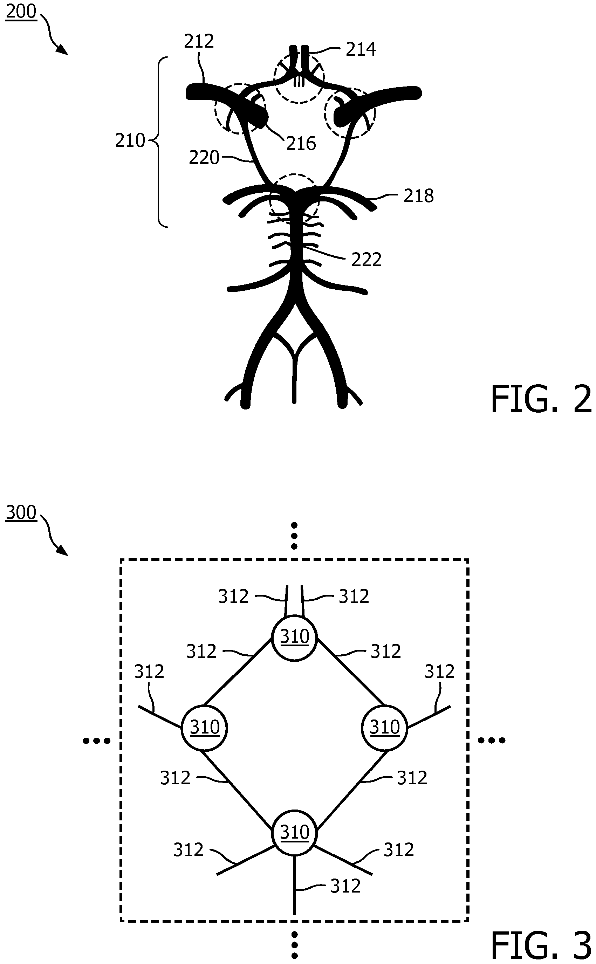

FIG. 2 is a schematic diagram illustrating a vasculature 200 of a patient's brain such as the brain 102, according to aspects of the present disclosure. The vasculature 200 may be imaged by an ultrasound imaging probe such as the probe 120. The blood flow (e.g., velocity and direction) within the vasculature 200 may be determined based on color-Doppler flow measurements, as described in greater detail herein. As shown, the vasculature 200 includes a ring-like arterial structure 210, which may be referred to as the CoW. The vasculature 200 is located at the base of a patient's brain. The vasculature 200 includes a network of blood vessels. The vasculature 200 may supply blood to the brain and surrounding tissues and structures. As shown, the vasculature 200 includes six major arteries including an ACA 214, an ICA 216, an MCA 212, a PCA 218, a posterior communicating artery 220, and a BA 222. Each of the arteries 212, 214, 216, 218, 220, and 222 may branch into smaller vessels. As described above, the blood vessel arrangement around the CoW may be substantially similar for all patients. Thus, the structural arrangement (e.g., the connectivity, topology and/or locations) of the arteries 212, 214, 216, 218, 220, and 222 may be described in a graphical representation and used for constructing a cerebrovascular atlas 140 as described in greater detail herein.

FIG. 3 is a schematic diagram illustrating a graphical representation 300 of a portion of a vasculature of a patient's brain, according to aspects of the present disclosure. For example, the graphical representation 300 corresponds to a portion (e.g., around the structure 210) of the vasculature 200. The graphical representation 300 represents the structural arrangement of blood vessels within the vasculature. The graphical representation 300 includes nodes 310 connected by edges 312 representing the geometric topology of blood vessels such as the vessels 104 and the arteries 212, 214, 216, 218, 220, and 222 in space. The nodes 310 may correspond to vessel bifurcations, endpoints of blood vessels, and/or vascular locations along segments or flow pathways of blood vessels. Each edge 312 may connect two or more nodes 310. For example, a blood vessel may be divided into multiple segments represented by a series of nodes 310 interconnected by edges 312. The graphical representation 300 can be referred to as a connectivity graph, a vessel tree, or a node-edge diagram.

As an example, the intersections of the arteries 212, 214, 216, 218, 220, and 222 as shown by the dotted circles in FIG. 2 are represented by the nodes 310 and the segments of the arteries 212, 214, 216, 218, 220, and 222 connecting to the intersections are represented by the edges 312. While FIG. 3 employ nodes 310 to represent vessel bifurcations, in some embodiments, a blood vessel (e.g., the MCA 212) may be represented by multiple nodes 310 interconnected by multiple edges 312 corresponding to segments of the blood vessel. Thus, the graphical representation 300 may include any suitable number of nodes 310 interconnected by any suitable number of edges 312. In addition, the graphical representation 300 can include nodes 310 and edges 312 representing smaller vessels that are fed by the major arteries 212, 214, 216, 218, 220, and 222.

In an embodiment, the nodes 310 and/or the edges 312 are represented by spatial Cartesian coordinates and/or flow vectors as described in greater detail herein. In an embodiment, the cerebrovascular atlas 140 describes connectivity, topology, and/or location information of blood vessels within human brains using the graphical representation 300. In an embodiment, the CNN 142 operates on a graphical representation 300 of a Doppler image as described in greater detail herein.

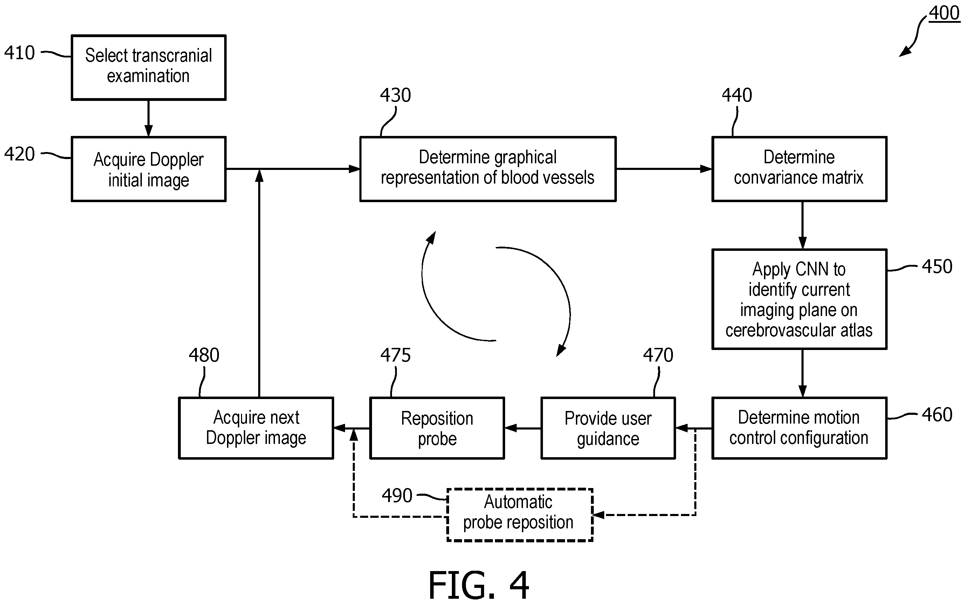

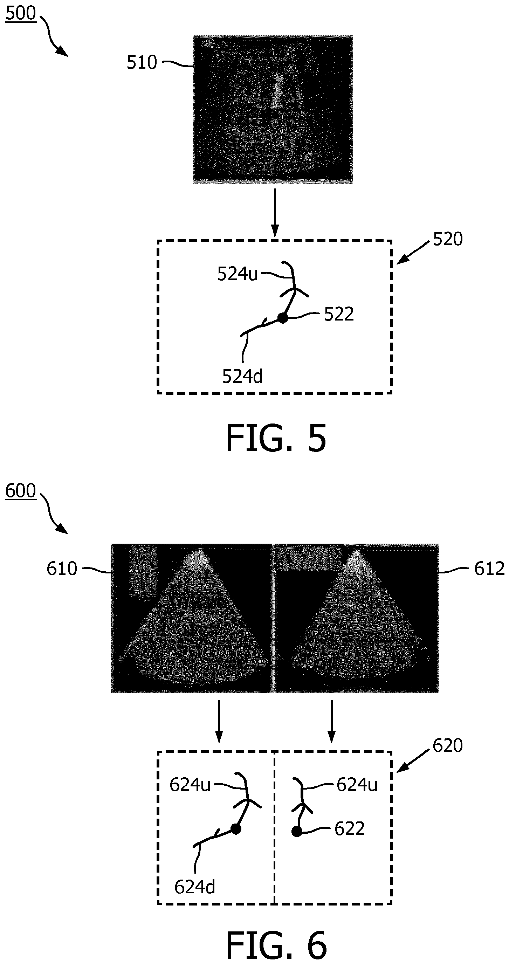

FIGS. 4-6 collectively illustrate a transcranial examination using the system 100. FIG. 4 is a schematic diagram illustrating a scheme 400 for guiding an ultrasound imaging probe to a desired imaging plane for a transcranial examination, according to aspects of the present disclosure. FIG. 5 illustrates an example of 2D Doppler imaging 500, according to aspects of the present disclosure. FIG. 6 illustrates an example of 3D Doppler imaging 600, according to aspects of the present disclosure. The scheme 400 may be implemented by the system 100. As illustrated, the scheme 400 includes a number of enumerated steps, but embodiments of the scheme 400 may include additional steps before, after, and in between the enumerated steps. In some embodiments, one or more of the enumerated steps may be omitted or performed in a different order.

At step 410, a user may select a transcranial examination for a patient, for example, based on potential pathology or a clinician-directed protocol. For example, the user may determine to examine a region near the MCA (e.g., a left M1 segment of an MCA 212), the PCA (e.g., a right segment of a PCA 218), the ICA (e.g., the ICA 216), the ACA (e.g., the ACA 214), the posterior communicating artery (e.g., the posterior communicating artery 220), the BA (e.g., the BA 222), or any region of interest within the patient's brain (e.g., the brain 102). The user may position the ultrasound imaging probe 120 adjacent to or in contact with the patient's head (e.g., the head 110) at an initial location proximal to a transcranial window for the selected transcranial examination. For example, the transcranial window may be a temporal transcranial window, a submandibular transcranial window, a suboccipital transcranial window, or any other suitable transcranial window.

In an embodiment, the scheme 400 may employ a UI (e.g., the UI 144) to guide the user in locating a suitable transcranial window for the selected transcranial examination. For example, the UI may display a brain map (e.g., the cerebrovascular atlas 140) and the user may select a desired vascular location for a transcranial examination from the brain map. Alternatively, the user may select a type of transcranial examination (e.g., an MCA examination). The processing component 136 may determine a transcranial window suitable for the desired transcranial examination based on the user's selection. The UI may provide indications and/or instructions to guide the user to the corresponding transcranial window. In addition, the processing component 136 may determine a target imaging plane for the selected vascular location or the selected transcranial examination.

At step 420, the user may acquire an initial Doppler image of the patient's head using the probe 120 while the probe 120 is at the initial location. The initial Doppler image may include blood flow measurements of the blood vessels within the patient's head. In one embodiment, the initial Doppler image may be a 2D color-Doppler image 510 as shown in FIG. 5. In another embodiment, for 3D color-Doppler imaging, multiple 2D Doppler images, for example, obtained from X-plane imaging can be used. X-Plane refers to a high frame rate 3D imaging strategy where two 2D planes are obtained at different angles about the axis of acoustic propagation, commonly 90 degrees. As an example, the initial Doppler image may include color-Doppler images 610 and 612 as shown in FIG. 6 corresponding to different views of a 3D image volume. In general, the scheme 400 can be applied to 2D input data, X-Plane input data, multiple-plane input data, which may or may not be separated by 90 degrees, or full 3D imaging data. As the size of the input data increases, the computational cost will increase, but the ability of the system to identify the proper location in the atlas also increases.

The probe 120 may emit ultrasound waves towards the patient's head, which then bounces off structures (e.g., brain tissues and vessels) within the patient's head and received by the probe 120 as echo signals. The probe 120 may be configured to emit ultrasound signals at a specific frequency (e.g., between about 1 MHz to about 3 MHz) depending on the desired imaging resolution and/or absorption of energy by the skull. The speed of the blood in relation to the probe causes a phase shift, with the frequency being increased or decreased (i.e., Doppler effect). For example, the processing component 136 at the host 130 may receive the echo signals, determine changes in the frequency, and calculate the velocity of scatterers.

In an embodiment, the processing component 136 can employ the following Doppler equation:

$$\Delta f = (2 \times f0 \times V \times \cos \theta))/C \qquad (1)$$

where $\Delta f$ is the frequency shift, f0 is the frequency of the transmitted wave, V is the velocity of the reflecting object (e.g.,

a red blood cell), θ is the angle between the incident wave and the direction of the movement of the reflecting object (i.e., the angle of incidence), and C is the velocity of sound in the medium. The frequency shift is maximal when the transducer is oriented parallel to the direction of the blood flow and the θ is zero degrees (cos 0 = 1). The frequency shift is absent when the transducer is oriented perpendicular to the direction of the blood flow and the θ is 90 degrees (cos 90 = 0). Higher Doppler frequency shifts are obtained when the velocity is increased, the incident wave is more aligned with the direction of blood flow, and/or when a higher frequency is emitted.

At step 430, the processing component 136 may determine a graphical representation of the blood vessels captured by the acquired Doppler images. For example, the Cartesian coordinates of the blood vessels may be graphically represented by nodes interconnected by edges as shown in the graphical representation 300 described above with respect to FIG. 3. For example, the processing component 136 may convert the color-Doppler image 510 into a graphical representation 520 including nodes 522 (e.g., the nodes 310) connected by edges 524 (e.g., the edges 312). For example, the edge 524u may represent an upstream blood flow and may be color-coded in red or indicated by a red arrow, while the edge 524d may represent a downstream blood flow and may be color-coded in blue or indicated by a blue arrow. The interconnections of the nodes 522 and the edges 524 in the graphical representation 520 may be expressed as a set of flow vectors. The orientation of a flow vector in space can be expressed as shown below:

$$V_i = \{x_i, y_i, \theta_i, \varphi_i\}, \tag{2}$$

where $V_i$ represents a flow vector $i$, $x_i$ and $y_i$ represent the x-coordinate and the y-coordinate, respectively, in a 2D ultrasound imaging plane, $\theta_i$ represents an elevation angle, and $\varphi_i$ represents an azimuthal angle.

Similarly, when the Doppler image corresponds to the 3D color-Doppler images 610 and 612, the processing component 136 may convert the 3D color-Doppler images 610 and 612 into a graphical representation 620 including nodes 622 (e.g., the nodes 310) connected by edges 624 (e.g., the edges 312). The edges 624u may represent an upstream blood flow and the edge 624d may represent a downstream blood flow. The interconnections of the nodes 622 and the edges 624 in the graphical representation 620 may be expressed as a set of flow vectors. The orientation of a flow vector in space can be expressed as shown below:

$$V_i = \{x_i, y_i, z_i, \theta_i, \varphi_i, \psi_i\}, \tag{3}$$

where $V_i$ represents a flow vector {i}, $x_i$, $y_i$, $z_i$ represent the x-coordinate, the y-coordinate, and the z-coordinate, respectively, in a 3D ultrasound imaging volume, $\theta_i$ represents an elevation angle, $\varphi_i$ represents an azimuth angle, and $\psi_i$ represents a connectivity parameter.

For example, the graphical representation of the blood vessels in the Doppler image may be divided into subsets of coordinates expressed as shown below:

$$M(x, y, \theta, \varphi) = \begin{bmatrix} x_1 & x_2 & \dots x_N \\ y_1 & y_2 & \dots y_N \\ z_1 & z_2 & \dots z_N \\ \theta_1 & \theta_2 & \dots \theta_N \\ \varphi_1 & \varphi_2 & \dots \varphi_N \\ \varphi_1 & \varphi_2 & \dots \varphi_N \end{bmatrix}. \tag{4}$$

The matrix $M$ includes a vectorized representation of the graphical representation of the blood vessel.

At step 440, the processing component 136 may determine a covariance matrix, denoted as C, as shown below:

$$C = M^T \times W \times M, \tag{5}$$

where $M^T$ represents the transpose of the matrix M and $W$ represents a weighting matrix including weighting factors for the coordinates. In other words, the covariance matrix $C$ includes the weighted inner product of the N subset of coordinates. The matrix $M$ may be within a data set $R^N$ with N subset of coordinates (e.g., $\in R^N$) and the covariance matrix $C$ may be within a data set $R^{N \times N}$ (e.g., $C \in R^{N \times N}$).

In an embodiment, the weighting factors may be empirically determined and can be different for each coordinate (e.g., between the duplet (x, y), and the duplet (θ, φ)). In an embodiment, the weighting factors in the matrix $W$ may be configured such that nodes (e.g., the nodes 310, 522, and 622) corresponding to main arteries are given a higher weight (e.g., a larger value) and the nodes corresponding vessel branches are given a smaller weight (e.g., a smaller value). The weighting factors may be determined manually for a transcranial examination. For example, for a MCA examination, the nodes associated with an MCA may be given higher weights than other blood vessels. In some embodiments, the weighting matrix W may be excluded from the computation of the covariance matrix C (e.g., all weighting factors are set to values of ones).

At step 450, the processing component 136 may apply the CNN 142 to the covariance matrix C to identify a current imaging plane of the probe 120 with respect to the cerebrovascular atlas 140. The internal architecture, the training, and the application of the CNN 142 are described in greater detail herein.

At step 460, the processing component 136 may determine a motion control configuration (e.g., including translation and rotation parameters) for repositioning the probe 120 to the target imaging plane for the selected transcranial examination. For example, the target imaging plane for the selected transcranial examination with respect to the cerebrovascular atlas 140 is predetermined. After the current imaging plane of the probe 120 is identified with respect to the cerebrovascular atlas 140, the motion control configuration to reach the target imaging plane may be determined based on a geometric distance (e.g., a translation) and/or angular (e.g., a rotation) computation.

In an embodiment, the processing component 136 may compute a rotation matrix between the current imaging plane and the target imaging plane to obtain angulation or rotation parameters, denoted as $(\theta, \varphi)$, for repositioning the probe 120 to point towards the target imaging plane or target field-of-view. If the rotation is not sufficient in reaching the target imaging plane, the processing component 136 may additionally compute a translation vector between the current imaging plane and the target imaging plane, which may be outside a current field-of-view.

At step 470, the display 134 may provide user guidance for repositioning the probe 120 to the target imaging plane. For example, the display 134 may display a graphical view of the probe 120 indicating an amount or direction of a translation and/or an amount or a direction of rotation for repositioning the probe 120. The graphical display may include an animated motion of the probe 120 to reach the target imaging plane. The display 134 may display a graphical view including an overlay of the current imaging plane and/or the target imaging plane on top of the cerebrovascular atlas 140. The graphical display is described in greater detail herein.

At step 475, the user may reposition the probe 120 according to the user guidance to a next location. At step 480, a next Doppler image may be acquired while the probe 120 is at the new location. In some embodiments, the steps 430-480 may be repeated for the probe 120 to reach the target imaging plane.

In some embodiments, the motion control configuration may be sent to a mechanical actuation unit (e.g., the robotic system 160) to automatically control or reposition the probe 120 as shown in the step 490 instead of providing user guidance and having the user to reposition the probe 120 as shown in steps 470 and 475.

After the probe 120 is aligned to the target imaging plane, the user may proceed with the selected transcranial examination. In some embodiments, the scheme 400 may further employ spectral Doppler to further classify the blood vessels under examination and provide further guidance to the user with a higher accuracy in reaching the target imaging plane. The coordinates of the desired or target blood vessels obtained from the CNN 142 may be input into a Doppler beamforming unit so that continuous Doppler traces, blood flow velocities can be generated. In some embodiments, after the transcranial examination is completed, the user may update the CNN 142 and/or the cerebrovascular atlas 140 with information (e.g., coordinates) associated with the target blood vessels.

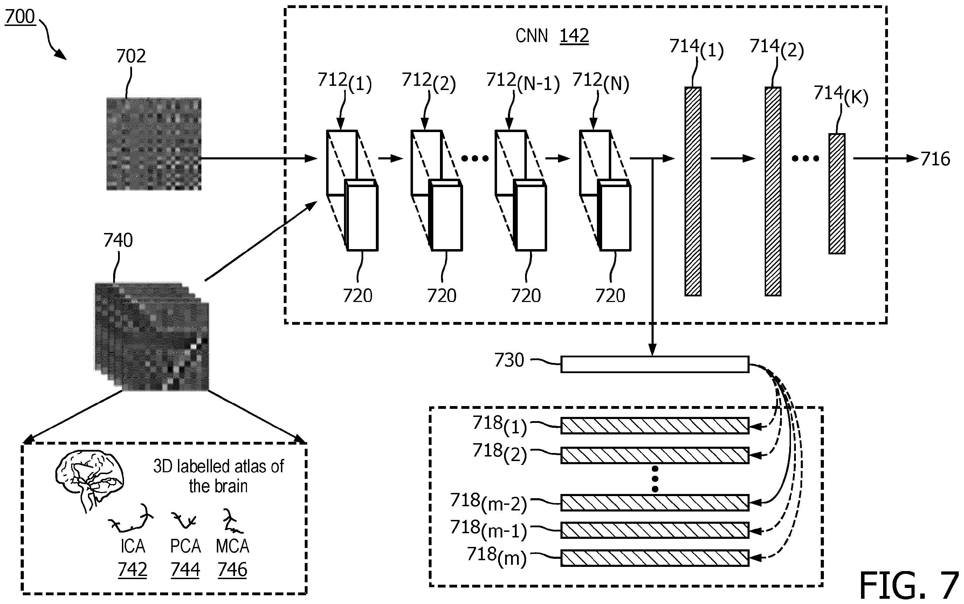

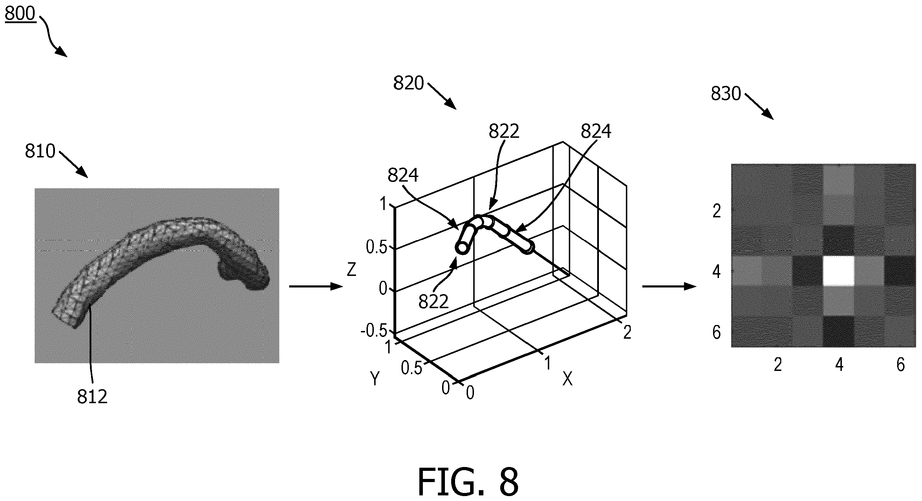

FIGS. 7-8 collectively illustrate mechanisms in employing the CNN 142 and the cerebrovascular atlas 140 for a transcranial examination. FIG. 7 is a schematic diagram illustrating a configuration 700 for the CNN 142, according to aspects of the present disclosure. FIG. 8 is a schematic diagram illustrating a scheme 800 for generating a covariance matrix from a cerebrovascular atlas, according to aspects of the present disclosure. The CNN 142 is trained using one or more cerebrovascular atlases 140 to identify a vascular location on a cerebrovascular atlas 140 given an input image. After the CNN 142 is trained, the CNN 142 is applied to a covariance image 702 (e.g., the covariance matrix C) computed in real-time from live imaging data during a transcranial examination, for example, as described in the step 450 of the scheme 400.

The CNN 142 may include a set of $N$ convolutional layers 712 followed by a set of $K$ fully connected layers 714, where $N$ and $K$ may be any positive integers. The values $N$ and $K$ may vary depending on the embodiments. In some embodiments, $N$ may be between about 3 to about 200 and $K$ may be between about 1 to about 5. Each convolutional layer 712 may include a set of filters 720 configured to extract imaging features (e.g., one-dimensional (1D) feature maps) from an input image. The fully connected layers 714 may be non-linear and may gradually shrink the high-dimensional output of the last convolutional layer $712_{(N)}$ to a length corresponding to the number of classification layers (e.g., various vascular locations on a cerebrovascular atlas 140) at the output 716 of the CNN 142. While not shown in FIG. 7, in some embodiments, the convolutional layers 712 may be interleaved with pooling layers, each including a set of downsampling operations that may reduce the dimensionality of the extracted imaging features. In addition, the convolutional layers 712 may include non-linearity functions (e.g., including rectified non-linear (ReLU) operations) configured to extract rectified feature maps.

During the training of the CNN 142, a cerebrovascular atlas 140 may be converted into coordinates or flow vectors represented by a matrix M as shown in Equation (4) above. In some embodiments, the coordinates and/or flow vectors may be stored in a 3D node file. The file may include additional information at each vertex or node (e.g., the nodes 310, 522, and 622) including an artery class, a flow direction, an artery diameter range, flow ranges (e.g., for an enddiastolic volume (EDV) and/or for an end-systolic volume (ESV)), and/or connectivity information (e.g., face and vertex).

The CNN 142 may be trained based on a weighted covariance C of the matrix M computed as shown in Equation (5) above. As described above, the cerebrovascular atlas 140 may include cerebrovascular topologies determined from real patient data that are obtained from clinical studies and/or live clinical data. The coordinates in the atlas 140 may be divided into subsets and labeled according to different locations of the brain, for example, including a subset 742 corresponding to an ICA region, a subset 744 corresponding to a PCA region, and a subset 746 corresponding to an MCA region. Each subset 742, 744, and 746 of the coordinates may be labeled according to corresponding vascular locations (e.g., an M1 segment of an MCA). A covariance matrix 740 may be computed for each subset 742, 744, and 746.

In an embodiment, a covariance matrix 740 may be generated as shown in FIG. 8. As shown in FIG. 8, a section of a PCA 812 in an atlas 810 (e.g., the atlas 140) is represented by a node diagram 820 (e.g., the representation 300) in space including nodes 822 (e.g., the nodes 310, 522, and 622) connected by edges 824 (e.g., the edges 312, 524, and 624). A covariance matrix 830 (e.g., the covariance matrix 740) computed from the node diagram 820.

The CNN 142 is trained on covariance matrices 740 of each subset 742, 744, and 746 retrieved from the atlas, for example, using forward and backward propagation. The coefficients of the filters 720 may be adjusted, for example, by using backward propagation to minimize the classification error (e.g., between a vascular location indicated by the output 716 and the label for the corresponding subset 742, 744, or 746). For example, the last convolutional layer $712_{(N)}$ may output a feature vector 718 with coordinates representing a particular vascular location and the output 716 may indicate a classification corresponding to the vascular location.

In an embodiment, the CNN 142 is trained to identify $m$ vascular locations (e.g., classifiers), where $m$ is a positive integer. Thus, the CNN 142 may produce an output 716 indicating one of the $m$ classes. For example, when the CNN 142 operates on the covariance matrix 740 of the subset 742, the CNN 142 may output a feature vector $718_{(1)}$ at the last convolutional layer $712_{(N)}$ and a classifier indicating an ICA at the output 716. Alternatively, when the CNN 142 operates on the covariance matrix 740 of the subset 746, the CNN 142 may output a feature vector $718_{(m-2)}$ at the last convolutional layer $712_{(N)}$ and a classifier indicating an MCA at the output 716. The training of the CNN 142 may be repeated using multiple cerebrovascular atlases 140 constructed from real patient data obtained via clinical studies, and/or life data from clinical settings.

During a transcranial examination, a covariance image 702 is inferred with the CNN 142 to (e.g., computed as shown in Equation (5)) in real-time based on ultrasound data obtained from imaging a patient's head (e.g., the head 110). The covariance image 702 is matched to corresponding labeled cerebral vessels in the cerebrovascular atlas 140 to estimate the likely vascular location within the patient's brain that the current frame of color-Doppler imaging represents. For example, the last convolutional layer $712_{(N)}$ may output a feature vector 730 to represent the input covariance image 702. The feature vector 730 may then be matched to the set of $m$ feature vectors 718 that were pre-generated by feeding the covariance matrices of $m$ labeled cerebrovascular atlases into the same CNN 142. The CNN 142 may indicate a classification of the feature vector 730 at the output 716 based on the matching of the feature vector 730 to the set of m labeled feature maps 718 as shown by the dotted curved arrows. As an example, the feature vector 730 may match the feature vector $718_{(m-2)}$ as shown by the solid curved arrows. Thus, the output 716 may indicate the classifier (e.g., the MCA) corresponding to the matched feature vector $718_{(m-2)}$. The matching of the feature vector 730 to the feature vector $718_{(m-2)}$ in turn identifies the vascular location of the current imaging plane corresponding to the covariance image 702 on the cerebrovascular atlas 140. The vascular location of the current imaging plane with respect to the cerebrovascular atlas 140 may be used to provide user guidance as described in greater detail herein.

In some embodiments, the CNN 142 may provide two possible matches at the output 716 for an acquired Doppler image. For example, the CNN 142 may output a match of about 50% for an MCA and a match of about 50% for an ICA. In such embodiments, the user may switch to configure the probe 120 to measure spectral Doppler to obtain velocity profiles to qualify the classification output by the CNN 142. For example, an additional CNN or other waveform matching techniques may be used to determine whether the acquired Doppler image corresponds to an image of an MCA or an image of an ICA. When using an additional CNN, the additional CNN may be trained based on velocity profiles of various vessels obtained from spectral Doppler. The additional CNN may have a substantially similar architecture as the CNN 142.

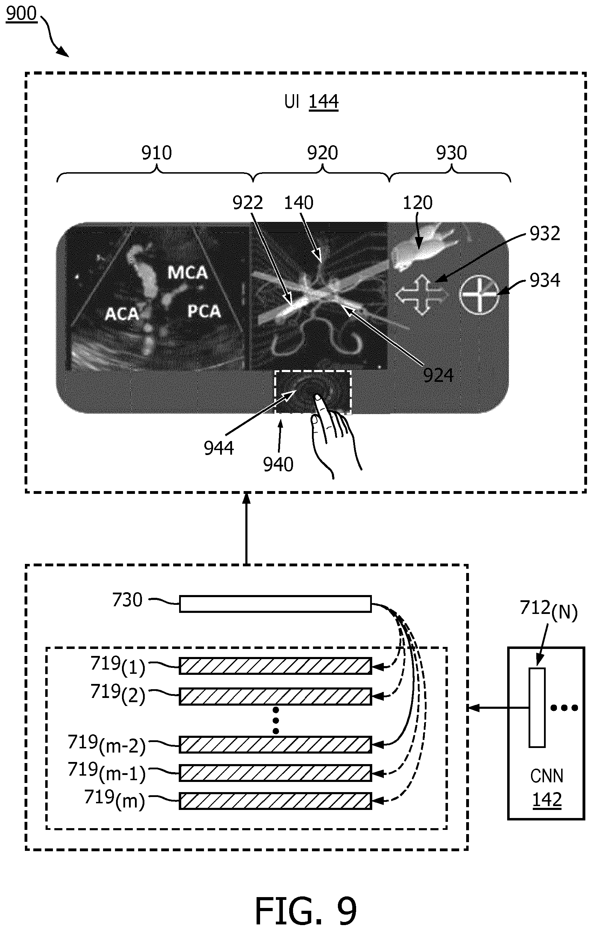

FIG. 9 is a schematic diagram illustrating a display view 900 for guiding transcranial ultrasound imaging, according to aspects of the present disclosure. The view 900 may correspond to a display view on the display 134 in the system 100. The view 900 includes three sub-views 910, 920, and 930. The sub-view 910, 920, and 930 may be displayed side-by-side as shown in FIG. 9 or alternatively configured in any suitable display configuration to provide similar functionalities.

The sub-view 910 shows a current image (e.g., the live color-Doppler images 510, 610, and 612) of vessels of a patient under an examination using the system 100. The current image may be captured by the probe 120 at a current imaging plane 922 in real-time. The current image may correspond to an image being input into the CNN 142 for computing the covariance image 702 in the configuration 700 described above with respect to FIG. 7. The sub-view 910 may include labels marking the vessels captured by the current image. As shown, the sub-view 910 includes labels marking an MCA (e.g., the MCA 212), an ACA (e.g., 214), and a PCA (e.g., 218).

The sub-view 920 shows an overlay of the current imaging plane 922 and a target imaging plane 924 (e.g., with partial transparency) based on the selected transcranial examination on top of a cerebrovascular topography (e.g., the cerebrovascular atlas 140). The overlay of the current imaging plane 922 may be based on a comparison of a feature vector 730 extracted from the current image against a set of $m$ feature vectors 718 extracted from cerebrovascular atlases 140. In some embodiments, the display of the cerebrovascular atlas 140 may be in 3D. The vessel under the current imaging and/or the target vessel for the transcranial examination may be highlighted on the cerebrovascular atlas 140.

The sub-view 930 provides a user with instructions to reposition the probe 120 from the current imaging plane 922 and to the target imaging plane 924 (e.g., determined in the step 460 of the scheme 400). As shown, the sub-view 930 may include a visual indicator 932 that may illustrate a required translation (e.g., based on a computed translation $(x, y)$) and a visual indicator 934 that may illustrate a required rotation (e.g., based on a computed rotation $(\theta, \varphi)$) for maneuvering the probe 120 to reach the target imaging plane 924. In some embodiments, the sub-view 930 may further display an animated view of the visual indicators 932 and 934 illustrating a suggested movement of the probe 120 to reach the target imaging plane 924.

In some embodiments, the UI 144 may further include a user interface portion 940, for example, including a dial 944. A user may configure the sub-view 920 by manipulating the dial 944. For example, when the target vessels for the transcranial examination is not within a current-field-of-view, the user may manipulate the dial 944 to increase the thickness of the imaging volume beyond the current field-of-view to obtain an expected view or a predicted virtual view of the target vessels. Thus, while the target vessels may not be in a current field-of-view, the sub-view 920 may allow a user to visualize the location of the target vessels with respect to the current imaging plane 922. In an embodiment, the virtual target vessels may be displayed in the sub-view 920 in a transparency mode. The virtual vessels correspond to vascular locations predicted by the CNN 142 based on the atlas 140. For example, the sub-view 920 may provide 3D location information of the target vessel while imaging is performed using 2D imaging. In some embodiments, the user interface portion 940 may include other buttons, slide bars, and/or any suitable user interface components that may accept user inputs.

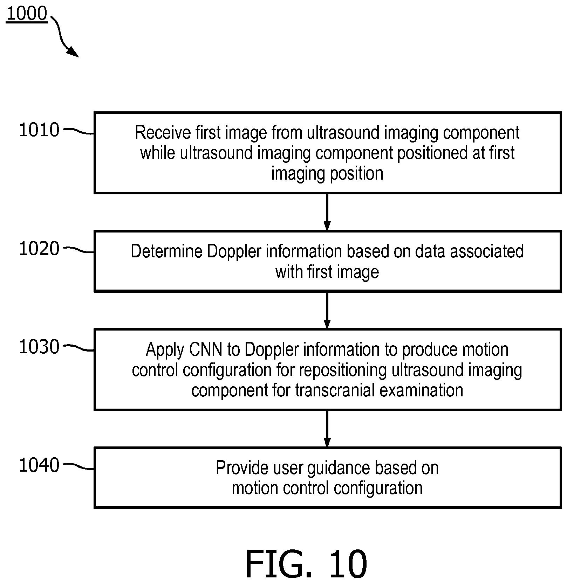

FIG. 10 is a flow diagram of a method 1000 of applying a CNN to guide an ultrasound imaging component to a desired imaging plane for a transcranial examination, according to aspects of the disclosure. Steps of the method 1000 can be executed by the system 100. The method 1000 may employ similar mechanisms as in the graphical representation 300, the scheme 400, and the CNN configuration 700 as described with respect to FIGS. 3, 4, and 7, respectively. As illustrated, the method 1000 includes a number of enumerated steps, but embodiments of the method 1000 may include additional steps before, after, and in between the enumerated steps. In some embodiments, one or more of the enumerated steps may be omitted or performed in a different order.

At step 1010, the method 1000 includes receiving a first image (e.g., the images 510, 610, and 612) from an ultrasound imaging component (e.g., the imaging component 122) while the ultrasound imaging component is positioned at a first imaging position with respect to the patient. The first image may be representative of blood vessels (e.g., the blood vessels 104 or the arteries 212, 214, 216, 218, 220, and 222 associated with CoW) of a brain (e.g., the brain 102) of a patient. The first imaging position may be any suitable location of the patient's head (e.g., the head 110). In some embodiments, the first imaging position may correspond to an imaging plane (e.g., the imaging plane 922).

At step 1020, the method 1000 includes determining Doppler information based on data associated with the first image. The Doppler information may be representative of blood flow within the blood vessels of the patient's brain. For example, the Doppler information may be computed using Equation (1) described above.

At step 1030, the method 1000 includes applying a CNN (e.g., the CNN 142) to the Doppler information to produce a motion control configuration for repositioning the ultrasound imaging component for a selected transcranial examination. The CNN may be trained based on at least a known blood vessel topography (e.g., the cerebrovascular atlas 140) within brains of a plurality of patients. In some embodiments, the known blood vessel topography may be determined based on a previous scanning of the brain of the patient under examination. The motion control configuration can include translation and/or rotation parameters for aligning the ultrasound imaging component to a target imaging plane (e.g., the target imaging plane 924) for the selected transcranial examination.

In some embodiments, the method 1000 may further include determining connectivity information (e.g., the matrix M) associated with the blood vessels of the patient's brain based on the Doppler information, determining a covariance matrix (e.g., the matrix C) based on the connectivity information and a weighting function (e.g., the matrix W), and applying the CNN to the covariance matrix. The connectivity information may be associated with the structural arrangement of the blood vessels and/or the flow pathways for blood flow through the blood vessels, for example, as shown in the graphical representation 300. The connectivity information may include coordinates (e.g., $\{x_i, y_i, \theta_i, \varphi_i\}$ shown in Equation (2) and $\{x_i, y_i, z_i, \theta_i, \varphi_i, \psi_i\}$ shown in Equation (3)) corresponding to vascular locations and flow pathways along the blood vessels of the patient's brain. The weighting function may be associated with a relevancy of the vascular locations or flow pathways with respect to the transcranial examination.

In some embodiments, the method 1000 may further include applying the CNN to the Doppler information to determine an imaging plane (e.g., the initial imaging plane 922) corresponding to the first imaging position within the known blood vessel topography and determining the motion control configuration based on the imaging plane and a target imaging plane (e.g., the target imaging plane 924) associated with the transcranial examination within the known blood vessel topography.

In some embodiments, the method 1000 may further include applying the CNN to the Doppler information to determine a feature vector (e.g., the feature vector 730) representative of the blood vessels of the patient's brain and determining the imaging plane within the known blood vessel topography based on a comparison of the feature vector against feature vectors (e.g., the feature maps 718) of the known blood vessel topography.

At step 1040, the method 1000 includes providing user guidance based on the motion control configuration. In some embodiments, the user guidance may include a display of an instruction, based on the motion control configuration, for operating the ultrasound imaging component such that the ultrasound imaging component is repositioned to the target imaging position, the instruction including at least one of a translation or a rotation of the ultrasound imaging component, for example, as shown by the visual indicators 932 and 934 in the sub-view 930. In some embodiment, the user guidance may include a display of a graphical view including an overlay of at least one of a first imaging plane associated with the first imaging position, a second imaging plane associated with the second imaging position, or the blood vessels of the patient's brain on top of the known blood vessel topography, for example, as shown in the sub-view 920. In some embodiments, the user guidance may include a display of a graphical view including an overlay of an expected view (e.g., a virtual out-of-plane view) of blood vessels of the patient's brain associated with the second imaging position on top of the known blood vessel topography.

Aspects of the present application can provide several benefits. For example, the use of deep learning to automatically identify a current imaging plane in real-time based on a current captured image and provide user guidance can eliminate the need for having a highly-experience operator to perform TCD ultrasound, and thus may expand the usage of TCD ultrasound in medical diagnostic procedures. In addition, the automatic identification and the user guidance can eliminate inter-operator variability in TCD ultrasound, and thus may provide more consistent and accurate results for TCD ultrasound-based examinations. For example, the disclosed embodiments can enable TCD ultrasound to be routinely performed in settings such as emergency rooms, rural medical centers, battlefields, and ambulances for continuous monitoring, triage, and evidence-based applications of therapy for conditions involving cerebrovasculature. The display of live Doppler images along with an overlay of the imaged vessels or the current imaging plane and a target imaging plane over a cerebrovascular map can provide further assistance in guiding the user to the target imaging plane. The real-time or live display of virtual vessels around a target vessel region outside a current field-of-view can provide further guidance to the user in searching or reaching the target vessels. Further, the real-time automatic identification enables continuous blood flow measurements without the need for a user to select a location for measurement within a field-of-view. While the disclosed embodiments are described in the context of training and applying predictive networks for guiding an ultrasound imaging probe, the disclosed embodiments can be applied to provide automatic alignments for any imaging component of any imaging modality.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the appended claims.

**Claims**

1.  A medical ultrasound imaging system (100) comprising:

    an interface (138) in communication with an ultrasound imaging component (122) and configured to receive a first image representative of blood vessels (104) of a brain (102) of a patient while the ultrasound imaging component is positioned at a first imaging position with respect to the patient;
    **characterized in that** the system further comprises a processing component (136) in communication with the interface (138) and configured to apply a convolutional network (142), CNN, to the first image to produce a motion control configuration for repositioning the ultrasound imaging component from the first imaging position to a second imaging position associated with a transcranial examination, the CNN trained based on at least a known blood vessel topography.

2. The system (100) of claim 1, wherein the processing component (136) is further configured to:

determine Doppler information representative of blood flow within the blood vessels (104) of the patient's brain (102) based on data associated with the first image, and
wherein the CNN (142) is applied to the Doppler information.

3. The system (100) of claim 2, wherein the processing component (136) is further configured to:

determine connectivity information associated with the blood vessels (104) of the patient's brain (102) based on the Doppler information, and
determine a covariance matrix (702) based on the connectivity information, and
wherein the CNN (142) is applied to the covariance matrix.

4. The system (100) of claim 3, wherein the connectivity information includes coordinates corresponding to vascular locations along the blood vessels (104) of the patient's brain (102).

5. The system (100) of claim 2, wherein the processing component (136) is further configured to:

apply the CNN (142) to the Doppler information to determine an imaging plane (922) corresponding to the first imaging position within the known blood vessel topography; and
determine the motion control configuration based on the imaging plane (922) and a target imaging plane (924) associated with the transcranial examination within the known blood vessel topography.

6. The system (100) of claim 5, wherein the processing component (136) is further configured to:

apply the CNN (142) to the Doppler information to determine a feature vector (730) representative of the blood vessels (104) of the patient's brain (102); and
determine the imaging plane (922) within the known blood vessel topography based on a comparison of the feature vector against the known blood vessel topography.

7. The system (100) of claim 1, wherein the CNN (142) is further trained based on at least a covariance matrix (740) determined based on connectivity information of the known blood vessel topography, and wherein the connectivity information includes coordinates corresponding to vascular locations along blood vessels indicated in the known blood vessel topography.

8. The system (100) of claim 1, wherein the motion control configuration includes at least one of a translation or a rotation of the ultrasound imaging component (122).

9. The system (100) of claim 1, further comprising a user interface (144) in communication with the processing component (136), the user interface configured to receive a selection of at least one of a type of the transcranial examination or a target vascular location associated with the transcranial examination, wherein the processing component is further configured to determine the second imaging position based on the selection.

10. The system (100) of claim 1, further comprising a display (134) in communication with the processing component (136), the display configured to display an instruction (932, 934), based on the motion control configuration, for operating the ultrasound imaging component (122) such that the ultrasound imaging component is repositioned to the second imaging position.

11. The system (100) of claim 1, further comprising a display (134) in communication with the processing component (136), the display configured to display a graphical view (900) including:

- an overlay of at least one of a first imaging plane (922) associated with the first imaging position, a second imaging plane (924) associated with the second imaging position, or the blood vessels of the patient's brain on top of the known blood vessel topography; or
- an overlay of an expected view of blood vessels of the patient's brain associated with the second imaging position on top of the known blood vessel topography.

12. A method (1000) of medical ultrasound imaging, comprising:

receiving (1010), from an ultrasound imaging component (122), a first image representative of blood vessels (104) of a brain (102) of a patient while the ultrasound imaging component is positioned at a first imaging position with respect to the patient;
**characterized in that** the method further comprises applying (1030) a convolutional network, CNN, (142) to the first image to produce a motion control configuration for repositioning the ultrasound imaging component (122) from the first imaging position to a second imaging position associated with a transcranial examination, the CNN trained based on at least a known blood vessel topography.

13. The method (1000) of claim 12, further comprising:

determining (1020) Doppler information representative of blood flow within the blood vessels of the patient's brain based on data associated with the first image,
wherein the CNN (142) is applied to the Doppler information.

14. The method (1000) of claim 13, further comprising:

determining connectivity information associated with the blood vessels (104) of the patient's brain (102) based on the Doppler information, the connectivity information including coordinates corresponding to vascular locations along the blood vessels of the patient's brain; and
determining a covariance matrix (702) based on the connectivity information, and
wherein the CNN (142) is applied to the covariance matrix.

15. The method (1000) of claim 12, further comprising:
transmitting (1040) an instruction to at least one of a display (134) or a robotic system (160), based on the motion control configuration, for operating the ultrasound imaging component (122) such that the ultrasound imaging component is repositioned to the second imaging position, the instruction including at least one of a translation or a rotation of the ultrasound imaging component.

**Patentansprüche**

1. Medizinisches Ultraschall-Bildgebungssystem (100), das Folgendes umfasst:

eine Schnittstelle (138), die mit einer Ultraschallabbildungskomponente (122) in Verbindung steht und so konfiguriert ist, dass sie ein erstes Bild empfängt, das Blutgefäße (104) eines Gehirns (102) eines Patienten darstellt, während die Ultraschallabbildungskomponente in einer ersten Abbildungsposition in Bezug auf den Patienten positioniert ist;
**dadurch gekennzeichnet, dass** das System ferner eine Verarbeitungskomponente (136) umfasst, die mit der Schnittstelle (138) in Verbindung steht und so konfiguriert ist, dass sie ein Faltungsnetzwerk (142), CNN, auf das erste Bild anwendet, um eine Bewegungssteuerungskonfiguration zum Neupositionieren der Ultraschallbildgebungskomponente von der ersten Bildgebungsposition zu einer zweiten Bildgebungsposition zu erzeugen, die mit einer transkraniellen Untersuchung verbunden ist, wobei das CNN auf der Grundlage mindestens einer bekannten Blutgefäßtopographie trainiert wird.

2. Das System (100) nach Anspruch 1, wobei die Verarbeitungskomponente(136) ferner konfiguriert ist, um:
Bestimmen von Doppler-Informationen, die für den Blutfluss in den Blutgefäßen (104) des Gehirns (102) des Patienten repräsentativ sind, auf der Grundlage von Daten, die dem ersten Bild zugeordnet sind, wobei das CNN (142) auf die Doppler-Informationen angewendet wird.

3. Das System (100) nach Anspruch 2, wobei die Verarbeitungskomponente(136) ferner konfiguriert ist, um:

Konnektivitätsinformationen zu bestimmen, die mit den Blutgefäßen (104) des Gehirns (102) des Patienten verbunden sind, basierend auf den Dopplerinformationen, und

Bestimmen einer Kovarianzmatrix (702) auf der Grundlage der Konnektivitätsinformationen, wobei das CNN (142) auf die Kovarianzmatrix angewendet wird.

4. System (100) nach Anspruch 3, wobei die Konnektivitätsinformationen Koordinaten enthalten, die den Gefäßpositionen entlang der Blutgefäße (104) des Gehirns (102) des Patienten entsprechen.

5. Das System (100) nach Anspruch 2, wobei die Verarbeitungskomponente (136) ferner konfiguriert ist, um:

den CNN (142) auf die Doppler-Informationen anzuwenden, um eine Abbildungsebene (922) zu bestimmen, die der ersten Abbildungsposition innerhalb der bekannten Blutgefäßtopographie entspricht; und

die Bewegungssteuerung auf der Grundlage der Abbildungsebene (922) und einer Zielabbildungsebene (924) zu bestimmen, die der transkraniellen Untersuchung innerhalb der bekannten Blutgefäßtopographie zugeordnet ist.

6. Das System (100) nach Anspruch 5, wobei die Verarbeitungskomponente (136) ferner konfiguriert ist, um:

den CNN (142) auf die Doppler-Informationen anzuwenden, um einen Merkmalsvektor (730) zu bestimmen, der für die Blutgefäße (104) des Gehirns (102) des Patienten repräsentativ ist; und

die Abbildungsebene (922) innerhalb der bekannten Blutgefäßtopographie auf der Grundlage eines Vergleichs des Merkmalsvektors mit der bekannten Blutgefäßtopographie zu bestimmen.

7. System (100) nach Anspruch 1, wobei das CNN (142) ferner auf der Grundlage mindestens einer Kovarianzmatrix (740) trainiert wird, die auf der Grundlage von Konnektivitätsinformationen der bekannten Blutgefäßtopographie bestimmt wird, wobei die Konnektivitätsinformationen Koordinaten enthalten, die Gefäßpositionen entlang von Blutgefäßen entsprechen, die in der bekannten Blutgefäßtopographie angegeben sind.

8. Das System (100) nach Anspruch 1, wobei die Bewegungssteuerungskonfiguration mindestens eine Übersetzungs- oder eine Drehung der Ultraschallbildgebungskomponente (122) umfasst.

9. System (100) nach Anspruch 1, das ferner eine Benutzerschnittstelle (144) umfasst, die mit der Verarbeitungskomponente (136) in Verbindung steht, wobei die Benutzerschnittstelle so konfiguriert ist, dass sie eine Auswahl von mindestens einer Art der transkraniellen Untersuchung oder einer vaskulären Zielstelle, die mit der transkraniellen Untersuchung verbunden ist, empfängt, wobei die Verarbeitungskomponente ferner so konfiguriert ist, dass sie die zweite Abbildungsposition auf der Grundlage der Auswahl bestimmt.

10. System (100) nach Anspruch 1, das ferner eine Anzeige (134) umfasst, die mit der Verarbeitungskomponente (136) in Verbindung steht, wobei die Anzeige so konfiguriert ist, dass sie auf der Grundlage der Bewegungssteuerungskonfiguration eine Anweisung (932, 934) anzeigt, um die Ultraschall-Bildgebungskomponente (122) so zu betreiben, dass die UltraschallBildgebungskomponente in die zweite Bildgebungsposition umpositioniert wird.

11. Das System (100) nach Anspruch 1 umfasst ferner eine Anzeige (134), die mit der Verarbeitungskomponente (136) in Verbindung steht, wobei die Anzeige so konfiguriert ist, dass sie eine grafische Ansicht (900) anzeigt, die Folgendes beinhaltet:

- eine Überlagerung von mindestens einer der folgenden Ebenen: eine erste Bildebene (922), die mit der ersten Bildgebungsposition verbunden ist, eine zweite Bildebene (924), die mit der zweiten Bildgebungsposition verbunden ist, oder die Blutgefäße des Gehirns des Patienten über der bekannten Blutgefäßtopographie; oder

- eine Überlagerung einer erwarteten Ansicht der Blutgefäße des Gehirns des Patienten, die der zweiten Bildgebungsposition zugeordnet ist, mit der bekannten Blutgefäßtopographie.

12. Verfahren (1000) zur medizinischen Ultraschallabbildung, das Folgendes umfasst:

Empfangen (1010) eines ersten Bildes von einer Ultraschallabbildungskomponente (122), das Blutgefäße (104) eines Gehirns (102) eines Patienten darstellt, während die Ultraschallabbildungskomponente in einer ersten Abbildungsposition in Bezug auf den Patienten positioniert ist;

**dadurch gekennzeichnet, dass** das Verfahren ferner das Anwenden (1030) eines Faltungsnetzwerks, CNN, (142) auf das erste Bild umfasst, um eine Bewegungssteuerungskonfiguration zum Neupositionieren der Ultraschallbildgebungskomponente (122) von der ersten Bildgebungsposition zu einer zweiten Bildgebungsposition, die mit einer transkraniellen Untersuchung verbunden ist, zu erzeugen, wobei das CNN auf der Grundlage mindestens einer bekannten Blutgefäßtopographie trainiert wird.

13. Das Verfahren (1000) nach Anspruch 12, das ferner Folgendes umfasst:
Bestimmen (1020) von Doppler-Informationen, die für den Blutfluss in den Blutgefäßen des Gehirns des Patienten repräsentativ sind, auf der Grundlage von Daten, die dem ersten Bild zugeordnet sind, wobei das CNN (142) auf die Doppler-Informationen angewendet wird.

14. Das Verfahren (1000) nach Anspruch 13, das ferner Folgendes umfasst:

Bestimmen von Konnektivitätsinformationen, die den Blutgefäßen (104) des Gehirns (102) des Patienten zugeordnet sind, auf der Grundlage der DopplerInformationen, wobei die Konnektivitätsinformationen Koordinaten enthalten, die den Gefäßpositionen entlang der Blutgefäße des Gehirns des Patienten entsprechen; und Bestimmen einer Kovarianzmatrix (702) auf der Grundlage der Konnektivitätsinformationen, wobei das CNN (142) auf die Kovarianzmatrix angewendet wird.

15. Das Verfahren (1000) nach Anspruch 12, das ferner Folgendes umfasst:
Übertragen (1040) einer Anweisung an mindestens eine Anzeige (134) oder ein Robotersystem (160), basierend auf der Bewegungssteuerungskonfiguration, zum Betreiben der Ultraschallabbildungskomponente (122), so dass die Ultraschallabbildungskomponente in die zweite Abbildungsposition umpositioniert wird, wobei die Anweisung mindestens eine Verschiebung oder eine Drehung der Ultraschallabbildungskomponente umfasst.

## Revendications

1. Système d'imagerie médicale ultrasonore (100) comprenant:

une interface (138) en communication avec un composant d'imagerie par ultrasons (122) et configurée pour recevoir une première image représentative des vaisseaux sanguins (104) d'un cerveau (102) d'un patient tandis que le composant d'imagerie ultrasonore est positionné à une première position d'imagerie par rapport au patient;
**caractérisé en ce que** le système comprend en outre un composant de traitement (136) en communication avec l'interface (138) et configuré pour appliquer un réseau convolutif (142), CNN, à la première image pour produire une configuration de commande de mouvement pour repositionner le composant d'imagerie ultrasonore de la première position d'imagerie à une deuxième position d'imagerie associée à un examen transcrânien, le CNN étant formé sur la base d'au moins une topographie connue des vaisseaux sanguins.

2. Système (100) selon la revendication 1, dans lequel le composant de traitement (136) est en outre configuré pour:

déterminer des informations Doppler représentatives du flux sanguin à l'intérieur des vaisseaux sanguins (104) du cerveau (102) du patient sur la base de données associées à la première image, et
dans lequel le CNN (142) est appliqué aux informations Doppler.

3. Système (100) selon la revendication 2, dans lequel le composant de traitement (136) est en outre configuré pour:
déterminer des informations de connectivité associées aux vaisseaux sanguins (104) du cerveau du patient (102) sur la base des informations Doppler, et déterminer une matrice de covariance (702) basée sur les informations de connectivité, et dans lequel le CNN (142) est appliqué à la matrice de covariance.

4. Système (100) selon la revendication 3, dans lequel les informations de connectivité comprennent des coordonnées correspondant à des emplacements vasculaires le long des vaisseaux sanguins (104) du cerveau (102) du patient.

5. Système (100) selon la revendication 2, dans lequel le composant de traitement (136) est en outre configuré pour:

appliquer le CNN (142) aux informations Doppler pour déterminer un plan d'imagerie (922) correspondant à la première position d'imagerie dans la topographie connue des vaisseaux sanguins; et

déterminer la configuration de commande de mouvement sur la base du plan d'imagerie (922) et d'un plan d'imagerie cible (924) associé à l'examen transcrânien dans la topographie connue des vaisseaux sanguins.

6. Système (100) selon la revendication 5, dans lequel le composant de traitement (136) est en outre configuré pour:

appliquer le CNN (142) aux informations Doppler pour déterminer un vecteur caractéristique (730) représentatif des vaisseaux sanguins (104) du cerveau (102) du patient; et
déterminer le plan d'imagerie (922) dans la topographie connue des vaisseaux sanguins sur la base d'une comparaison du vecteur caractéristique avec la topographie connue des vaisseaux sanguins.

7. Système (100) selon la revendication 1, dans lequel le CNN (142) est en outre entraîné sur la base d'au moins une matrice de covariance (740) déterminée sur la base d'informations de connectivité de la topographie de vaisseaux sanguins connue, et dans lequel les informations de connectivité comprennent des coordonnées correspondant à des emplacements vasculaires le long de vaisseaux sanguins indiqués dans la topographie de vaisseaux sanguins connue.

8. Système (100) selon la revendication 1, dans lequel la configuration de commande de mouvement comprend au moins l'une d'une translation ou d'une rotation du composant d'imagerie à ultrasons (122).

9. Système (100) selon la revendication 1, comprenant en outre une interface utilisateur (144) en communication avec le composant de traitement (136), l'interface utilisateur étant configurée pour recevoir une sélection d'au moins un type d'examen transcrânien ou un emplacement vasculaire cible associé à l'examen transcrânien, dans lequel le composant de traitement est en outre configuré pour déterminer la deuxième position d'imagerie sur la base de la sélection.

10. Système (100) selon la revendication 1, comprenant en outre un affichage (134) en communication avec le composant de traitement (136), l'affichage étant configuré pour afficher une instruction (932, 934), basée sur la configuration de commande de mouvement, pour faire fonctionner le composant d'imagerie par ultrasons (122) de telle sorte que le composant d'imagerie par ultrasons soit repositionné dans la deuxième position d'imagerie.

11. Système (100) selon la revendication 1, comprenant en outre un affichage (134) en communication avec le composant de traitement (136), l'affichage étant configuré pour afficher une vue graphique (900) comprenant:

- une superposition d'au moins l'un d'un premier plan d'imagerie (922) associé à la première position d'imagerie, d'un deuxième plan d'imagerie (924) associé à la deuxième position d'imagerie, ou des vaisseaux sanguins du cerveau du patient par-dessus la topographie connue des vaisseaux sanguins; ou
- une superposition d'une vue attendue des vaisseaux sanguins du cerveau du patient associée à la deuxième position d'imagerie par-dessus la topographie connue des vaisseaux sanguins.

12. Procédé (1000) d'imagerie médicale ultrasonore, comprenant:

la réception (1010), en provenance d'un composant d'imagerie par ultrasons (122), d'une première image représentative de vaisseaux sanguins (104) d'un cerveau (102) d'un patient tandis que le composant d'imagerie par ultrasons est positionné à une première position d'imagerie par rapport au patient;
**caractérisé en ce que** le procédé comprend en outre l'application (1030) d'un réseau convolutionnel, CNN, (142) à la première image pour produire une configuration de commande de mouvement pour repositionner le composant d'imagerie par ultrasons (122) de la première position d'imagerie à une deuxième position d'imagerie associée à un examen transcrânien, le CNN étant formé sur la base d'au moins une topographie connue des vaisseaux sanguins.

13. Procédé (1000) selon la revendication 12, comprenant en outre:
la détermination (1020) d'informations Doppler représentatives du flux sanguin à l'intérieur des vaisseaux sanguins du cerveau du patient sur la base des données associées à la première image, dans laquelle le CNN (142) est appliqué aux informations Doppler.

14. Procédé (1000) selon la revendication 13, comprenant en outre:

déterminer des informations de connectivité associées aux vaisseaux sanguins (104) du cerveau du patient (102) sur la base des informations Doppler, les informations de connectivité comprenant des coordonnées correspondant à des emplacements vasculaires le long des vaisseaux sanguins du cerveau du patient; et déterminer une matrice de covariance (702) basée sur les informations de connectivité, et dans lequel le CNN (142) est appliqué à la matrice de covariance.

**15.** Procédé (1000) selon la revendication 12, comprenant en outre:
la transmission (1040) d'une instruction à au moins l'un d'un affichage (134) ou d'un système robotique (160), sur la base de la configuration de commande de mouvement, pour faire fonctionner le composant d'imagerie par ultrasons (122) de sorte que le composant d'imagerie par ultrasons soit repositionné dans la deuxième position d'imagerie, l'instruction comprenant au moins l'une d'une translation ou d'une rotation du composant d'imagerie par ultrasons.

FIG. 1

FIG. 2

FIG. 3

FIG. 4

EP 3 742 979 B1

500

510

520

524u

522

524d

FIG. 5

600

610

612

620

624u

624u

622

624d

FIG. 6

FIG. 7

FIG. 8

FIG. 9

1000

1010 — Receive first image from ultrasound imaging component while ultrasound imaging component positioned at first imaging position

1020 — Determine Doppler information based on data associated with first image

1030 — Apply CNN to Doppler information to produce motion control configuration for repositioning ultrasound imaging component for transcranial examination

1040 — Provide user guidance based on motion control configuration

# FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020103436 A